# EUROPEAN PATENT APPLICATION

(11) **EP 2 351 641 A1**
(43) Date of publication of application: **03.08.2011**
(21) Application number: 09817770.2
(22) Date of filing: 29.09.2009
(51) Int. Cl.: B29C 47/06, A61M 25/00, B29C 47/92

(54) **MULTILAYER COATING EQUIPMENT, AND MULTILAYER COATING METHOD**

(30) Priority: 30.09.2008 JP 2008254646
(71) Applicant: FUJIFILM Corporation, Tokyo 106-8620 (JP)
(72) Inventor: MIYASAKA, Rei, Fujinomiya-shi Shizuoka 418-8666 (JP); TAMAKI, Kenichirou, Odawara-shi Kanagawa 250-0001 (JP)
(74) Representative: Höhfeld, Jochen
(86) International application number: PCT/JP2009/066950
(87) International publication number: WO 2010/038738

(57) **Abstract**

Provided are a multilayer coating apparatus and a multilayer coating method in which an uneven flow can be reduced when two types of resins having different softening points are caused to join together and a wire member is coated therewith. A coating apparatus (10) includes: a first extruder (12) and a second extruder (14); a crosshead (16) for coating a wire member with a resin layer with a double-layer structure; a first nozzle (18) which connects the first extruder (12) and the crosshead (16) to each other; a second nozzle 20 which connects the second extruder (14) and the crosshead (16) to each other; a control unit 22 which controls supply amounts of the first extruder (12) and the second extruder (14); and a heater (24) which heats the second nozzle (20). The second nozzle (20) is longer than the first nozzle (18).

## Description

### Technical Field

The present invention relates to a multilayer coating apparatus and a multilayer coating method, and more particularly, to a multilayer coating apparatus and a multilayer coating method which are suitable to manufacture a flexible tube constituting an insertion part of an endoscope.

### Background Art

In general, an endoscope includes a hand operation part and an insertion part provided in connection with the hand operation part. The hand operation part is gripped by an operator, and the insertion part is inserted into the body of a subject.

The insertion part includes a flexible tube part, a bending part, and a leading end part in the stated order from the hand operation part side. The leading end part is provided with an observation optical system including a lens and a prism. A forceps channel for inserting a treatment tool therethrough, a bending wire, a light guide, a signal cable, and the like are inserted through the flexible tube part.

The flexible tube which is a main component constituting the insertion part of the endoscope includes: a spiral tube which is formed by winding a metal belt material in a spiral manner; a tubular net body which covers the spiral tube; and an outer coating layer of urethane resin or the like which is laminated on a surface of the tubular net body. The insertion part is required to have a higher flexibility at the leading end part thereof in order to facilitate the insertion into a body, and have a lower flexibility and a higher stiffness on the hand operation part side of the flexible tube part thereof.

In order to fulfill the above-mentioned requirement, PLT 1 describes a flexible tube including an outer coating layer with a double-layer structure of a soft resin layer and a hard resin layer. The outer coating layer is formed by using two types of resins of hard and soft resins so that the ratio of the soft resin is increased on the leading end side whereas the ratio of the hard resin is increased on the hand operation part side.

### Citation List

### Patent Literature

PTL 1: Japanese Utility Model Application Laid-Open No. 55-231919

### Summary of Invention

### Technical Problem

Incidentally, in the formation of the outer coating layer of PLT 1, the ratio of the thicknesses of the soft resin layer and the hard resin layer is changed while the total thickness of the layers is kept substantially constant, and in this state, the outer coating layer with the double-layer structure is formed on a wire member such as a metal spiral tube. In order to change the ratio of the thicknesses of the soft resin and the hard resin, the soft resin and the hard resin are caused to join together for coating while the flow rates thereof are changed. Therefore, there has been a problem that an uneven flow is generated in the outer coating layer with the double-layer structure of the soft resin and the hard resin or at an interface between the two layers.

The present invention has been made in view of the above-mentioned circumstances, and therefore has an object to provide a multilayer coating apparatus and a multilayer coating method in which an uneven flow is less likely to be generated when two types of resins having different softening points are caused to join together and a wire member is coated therewith.

### Solution to Problem

As a result of intensive studies, the inventors of the present invention found out that, when two types of resins having different softening points are caused to join together and a wire member is coated therewith, an uneven flow can be reduced by minimizing a difference in viscosity between the two resins.

In order to achieve the above-mentioned object, a multilayer coating apparatus which coats a wire member with a plurality of resin layers, comprising: a first extruder which supplies a first resin; a first nozzle which is connected to an exit of the first extruder; a second extruder which supplies a second resin having a softening point different from a softening point of the first resin; a second nozzle which is connected to an exit of the second extruder and is longer than the first nozzle; a crosshead which is connected to the first nozzle and the second nozzle and coats the wire member with a first resin layer and a second resin layer; a control unit which controls supply amounts of the first extruder and the second extruder so that a total thickness of the first resin layer and the second resin layer used for the coating is substantially constant and a ratio of a thickness of the first resin layer to a thickness of the second resin layer gradually changes; and a heater which heats the second nozzle.

In the above-mentioned multilayer coating apparatus, the second nozzle is made longer than the first nozzle, and the second nozzle is provided with the heater and is heated so that the temperature of the second nozzle is higher than the temperature of the second extruder. With this configuration, the viscosity of the second resin can be adjusted to substantially the same value as the viscosity of the first resin while the second resin is supplied to the crosshead. The first resin and the second resin join together at substantially the same viscosity, and then the wire member is coated therewith. Accordingly, the uneven flow can be prevented.

Generally, in the case where the length of a nozzle is small, the resin temperature and the resin viscosity when the resin flows into a crosshead are the same as the resin temperature and the resin viscosity at the exit of an extruder. When the rotation speed of the extruder is low, the resin temperature is low, and the resin viscosity is high. On the other hand, when the rotation speed of the extruder is high, the resin temperature is high, and the resin viscosity is low.

In the present invention, the length of the second nozzle is large, and the second nozzle is provided with the heater. The second nozzle is heated by the heater, whereby the resin passing through the second nozzle can be heated. When the rotation speed of the second extruder is low, the temperature of the second resin at the exit of the second extruder is low, and the resin viscosity is high. However, since the speed of the second resin passing through the second nozzle is low and the length of the second nozzle is large, the amount of heat which is applied to the second resin per unit time due to a heat transfer effect from the heater is large. Accordingly, the temperature of the second resin flowing into the crosshead can be made higher, and the viscosity thereof can be made lower. With this configuration, the viscosities of the first resin and the second resin flowing into the crosshead can be made substantially the same. As a result, the uneven flow can be prevented.

In the above-mentioned multilayer coating apparatus, it is preferable that the softening point of the second resin be lower than the softening point of the first resin.

The above-mentioned multilayer coating apparatus may further include a feeder which continuously feeds the wire member. The control device may control the first extruder and the second extruder in accordance with a pattern for gradually changing the ratio of the thickness of the first resin layer to the thickness of the second resin layer, so as to repeat the pattern. The wire member may at least include: a plurality of coating target members; one dummy member; and a plurality of joint members for alternately coupling the coating target member and the dummy member to each other. In this case, the wire member is coupled so that, during a period when the control device executes one predetermined pattern, one of the plurality of coating target members is fed, and during a period from an end of the one predetermined pattern to a return to an initial state, the plurality of joint members and the one dummy member are fed.

Accordingly, during the period from the end of one pattern for coating the coating target member to the return to the initial state, that is, during a period when the wire member cannot be coated with the resin layer at a desired ratio, the dummy member can be fed. This makes it possible to prevent waste of materials.

Here, a length of each of the plurality of dummy members is decided in accordance with a feeding speed at which the feeder feeds the wire member and a length of the period from the end of the one pattern to the return to the initial state.

In order to achieve the above-mentioned object, a multilayer coating method according to another aspect of the present invention is a multilayer coating method for coating a wire member with a plurality of resin layers, including: a step of feeding the wire member to a crosshead; a first resin supply step of supplying a first resin from a first extruder to the crosshead via a first nozzle; a second resin supply step of supplying a second resin having a softening point different from a softening point of the first resin from a second extruder to the crosshead via a second nozzle being longer than the first nozzle; a coating step of coating the wire member with a first resin layer and a second resin layer having a softening point different from a softening point of the first resin layer by means of the crosshead so that a total thickness of the first resin layer and the second resin layer is substantially constant and a ratio of a thickness of the first resin layer to a thickness of the second resin layer gradually changes; and a heat step of heating the second nozzle.

In the above-mentioned multilayer coating method, it is preferable that the softening point of the second resin be lower than the softening point of the first resin.

In addition, in the above-mentioned multilayer coating method, it is preferable that the heat step include gradually increasing an amount of heat applied to the second resin layer per unit time as the ratio of the thickness of the second resin layer gradually becomes lower.

In addition, the above-mentioned multilayer coating method may further include a wire member feed step of continuously feeding the wire member to the multilayer coating apparatus. The wire member may at least include: a plurality of coating target members; one dummy member; and a plurality of joint members for alternately coupling the coating target member and the dummy member to each other. The coating step may include using a pattern for gradually changing the ratio of the thickness of the first resin layer to the thickness of the second resin layer, and repeating the pattern, to thereby coat the plurality of coating target members. The feed step may include feeding one of the plurality of coating target members during a period when one predetermined pattern is executed, and feeding the plurality of joint members and the one dummy member during a period from an end of the one predetermined pattern to a return to an initial state.

### Advantageous Effect of Invention

According to the present invention, it is possible to reduce an uneven flow when two types of resins having different softening points are caused to join together and a wire member is coated therewith.

### Brief Description of Drawings

Figure 1 is a schematic configuration view illustrating a coating apparatus according to the present embodiment.
Figure 2 is a partial cross sectional view illustrating a configuration of a flexible tube in an endoscope.
Figure 3 is a partial cross sectional view illustrating a configuration of a coupled flexible tube assembly.
Figure 4 is a schematic view illustrating a configuration of continuous formation equipment.
Figure 5 is a graph showing a relation between a rotation speed of each extruder and time.
Figure 6 is a graph showing a relation between a temperature and a melt viscosity of each conventional resin when the resins join together.
Figure 7 is a graph showing a relation between a temperature and a melt viscosity of each resin when the resins join together according to the present invention.

### Description of Embodiment

Hereinafter, a preferred embodiment of the present invention is described with reference to the attached drawings. The present invention is described by way of the following preferred embodiment, and can be changed according to a large number of methods without departing from the scope of the present invention, and embodiments other than the present embodiment can be adopted. Accordingly, all the changes within the scope of the present invention are encompassed in CLAIMS.

In addition, a numerical value range represented by using "to" in DESCRIPTION means the range including numerical values given before and after "to".

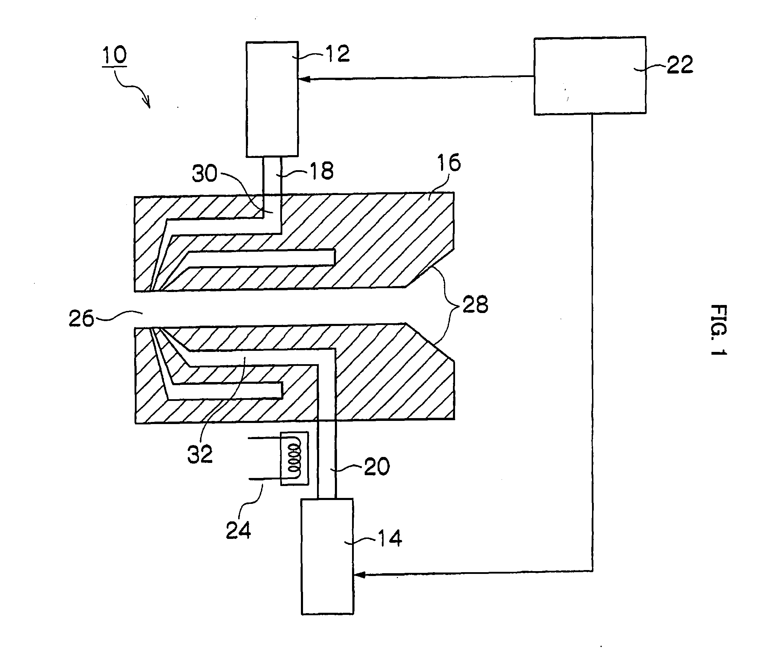

Figure 1 is a schematic configuration view illustrating a coating apparatus according to the present invention. A coating apparatus 10 includes: a first extruder 12 and a second extruder 14; a crosshead 16 for coating a wire member with a resin layer with a double-layer structure; a first nozzle 18 which connects the first extruder 12 and the crosshead 16 to each other; a second nozzle 20 which connects the second extruder 14 and the crosshead 16 to each other; and a control unit 22 which controls supply amounts of the first extruder 12 and the second extruder 14.

In the coating apparatus 10 according to the present embodiment, the length of the second nozzle 20 is larger than the length of the first nozzle 18, and a heater 24 is provided for heating the second nozzle 20.

The first extruder 12 and the second extruder 14 each include a hopper (not shown) and a screw (not shown). After the first extruder 12 and the second extruder 14 have been preheated to a predetermined temperature, resins are supplied to the first extruder 12 and the second extruder 14 from the respective hoppers by rotating the screws. In the first extruder 12 and the second extruder 14, the resins are fed in the directions of the first nozzle 18 and the second nozzle 20 while being plasticized by the screws.

In the present embodiment, resins having different softening points are supplied respectively to the first extruder 12 and the second extruder 14. A first resin (hard resin) having a higher softening point is supplied to the first extruder 12, and a second resin (soft resin) having a lower softening point is supplied to the second extruder 14.

The first nozzle 18 and the second nozzle 20 are connected to the crosshead 16. The resins fed from the first extruder 12 and the second extruder 14 are supplied to the crosshead 16 via the first nozzle 18 and the second nozzle 20. The second nozzle 20 is longer than the first nozzle 18. The heater 24 is provided for the second nozzle 20, whereby the second nozzle 20 is heated so as to have a temperature higher than a temperature at the exit of the second extruder 14.

A through passage 26 for guiding the wire member is formed at substantially the center of the crosshead 16. A tapered part 28, which is formed so as to be connected to the through passage 26, is provided at the rear end of the crosshead 16 corresponding to the entrance of the wire member. The tapered part 28 guides the insertion of the wire member into the through passage 26.

The crosshead 16 includes a first supply passage 30 and a second supply passage 32 for supplying the first resin and the second resin fed from the first extruder 12 and the second extruder 14, to the wire member. The first nozzle 18 and the second nozzle 20 described above are connected to the first supply passage 30 and the second supply passage 32, respectively. The first supply passage 30 and the second supply passage 32 are communicated from the first nozzle 18 side and the second nozzle 20 side to the vicinity of the exit of the wire member in the through passage 26.

In the crosshead 16, a hard resin layer and a soft resin layer are formed on the wire member while the wire member is caused to run inside of the through passage 26.

The resin supplied from the first supply passage 30 forms an outer layer (upper layer) on the wire member, and the resin supplied from the second supply passage 32 forms an inner layer (lower layer) on the wire member. Accordingly, in the present embodiment, an outer coating layer with a double-layer structure in which the outer layer is the hard resin layer and the inner layer is the soft resin layer is formed on the outer surface of the wire member.

The control unit 22 controls the supply amounts of the first extruder 12 and the second extruder 14. When the supply amount from the extruder is to be increased, the rotation speed of the screw is increased. When the supply amount from the extruder is to be reduced, the rotation speed of the screw is reduced. The film thicknesses of the hard resin layer and the soft resin layer in the outer coating layer are decided by controlling the supply amounts from the first extruder 12 and the second extruder 14.

The control unit 22 controls the supply amounts from the first extruder 12 and the second extruder 14 so that the total thickness of the hard resin layer and the soft resin layer coating the wire member is substantially constant and that the ratio of the thicknesses of the hard resin layer and the soft resin layer is gradually changed in synchronization with the running wire member. For example, the wire member is coated with the outer coating layer so that, first, the hard resin layer is thicker, and the soft resin layer is thinner, then, the ratio is gradually changed, and finally, the hard resin layer becomes thinner, and the soft resin layer becomes thicker. This constitutes one coating pattern. Coating based on this pattern (coating pattern) is repeatedly performed on the running wire member.

Next, description is given of a method of forming the outer coating layer with the double-layer structure on the wire member by using the coating apparatus 10. It should be noted that, in the present embodiment, a flexible tube used in an endoscope is described as an example of the wire member coated with the outer coating layer.

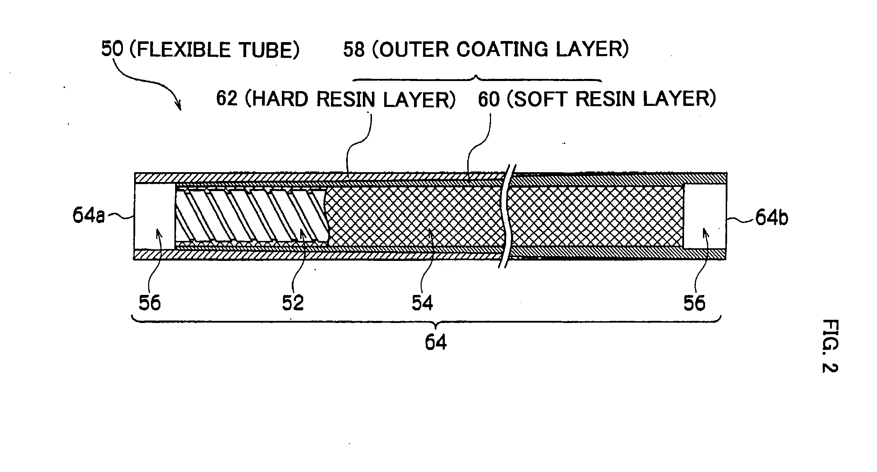

The configuration of the flexible tube is first described. Figure 2 is an enlarged partial cross sectional view illustrating the flexible tube constituting a flexible tube part of the endoscope. The flexible tube 50 includes: a spiral tube 52 which is formed by winding a metal belt material in a spiral manner at the innermost part; a tubular net body 54 which coats the spiral tube 52 and is formed by netting metal wires; caps 56 provided at both ends; and an outer coating layer 58 formed of a soft resin layer 60 and a hard resin layer 62. Further, the outer coating layer 58 is coated with a coat film (not shown) which contains, for example, silicon and has chemical resistance.

It should be noted that the spiral tube 52, the tubular net body 54, and the caps 56 provided at both ends constitute a flexible tube assembly 64. The flexible tube assembly 64 is a part of the wire member to be coated with the outer coating layer.

It should be noted that description is given hereinafter assuming that a distal end of the flexible tube assembly 64 is denoted by 64a and a proximal end of the flexible tube assembly 64 is denoted by 64b.

In the outer coating layer 58 of the present embodiment, the soft resin layer 60 constitutes a lower layer, and the hard resin layer 62 constitutes an upper layer. The hard resin layer 62 as the upper layer is formed so as to be thick on the distal end 64a side of the flexible tube assembly 64, and is formed so as to become gradually thinner from the distal end 64a to the proximal end 64b.

On the other hand, the soft resin layer 60 as the lower layer is formed so as to be thin on the distal end 64a side of the flexible tube assembly 64, and is formed so as to become gradually thicker from the distal end 64a to the proximal end 64b. The outer coating layer 58 is formed so that the total thickness of the hard resin layer 62 and the soft resin layer 60 is substantially constant.

In this way, the flexible tube 50 has a structure in which the flexibility is low and the stiffness is high on the distal end 64a side, and the flexibility is high on the proximal end 64b side. In an actual endoscope, the hand operation part is connected to the distal end 64a side of the flexible tube 50, and the bending part is connected to the proximal end 64b side of the flexible tube 50.

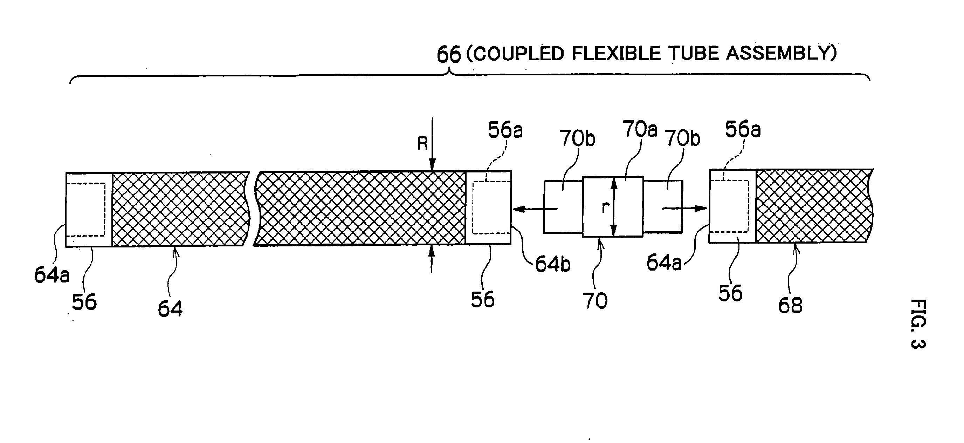

Figure 3 illustrates a coupled flexible tube assembly 66 which is configured as one wire member by coupling a plurality of the flexible tube assemblies 64 to each other. Before the formation of the outer coating layer 58, the plurality of flexible tube assemblies 64 are each connected to a dummy member 68 via a joint member 70. The joint member 70 includes a main body part 70a and coupling parts 70b provided on both sides of the main body part 70a. One of the coupling parts 70b is inserted into an inner circumferential surface 56a of the cap 56 which is provided to the proximal end 64b and is indicated by a dotted line. The other of the coupling parts 70b is inserted into an inner circumferential surface 56a of the cap 56 which is provided to one end of the dummy member 68 and is indicated by a dotted line. Further, the coupling part 70b on one end of the joint member 70 is inserted into the inner circumferential surface of the cap 56 on the other end of the dummy member 68. The coupling part 70b on the other end of the joint member 70 is inserted into the inner circumferential surface of the cap 56 provided to the distal end 64a. The coupled flexible tube assembly 66 is assembled by repeatedly connecting the flexible tube assembly 64, the joint member 70, the dummy member 68, and the joint member 70 in the stated order. The coupled flexible tube assembly 66 is supplied as the wire member to the above-mentioned coating apparatus. The coupled flexible tube assembly 66 is coated with the outer coating layer with the double-layer structure.

An outer diameter r of the main body part 70a of the joint member 70 is smaller than an outer diameter R of the flexible tube assembly 64. It should be noted that the present invention is not limited to this configuration, and the outer diameter of at least a part of the joint member 70 may be smaller than the outer diameter of the flexible tube assembly 64. In addition, the surface of the joint member 70 is coated with a separating agent of Teflon (registered trademark) or the like. With this configuration, after the outer coating layer 58 has been formed on the coupled flexible tube assembly 66, the outer coating layer 58 can be easily separated from the outer circumferential surface of the joint member 70.

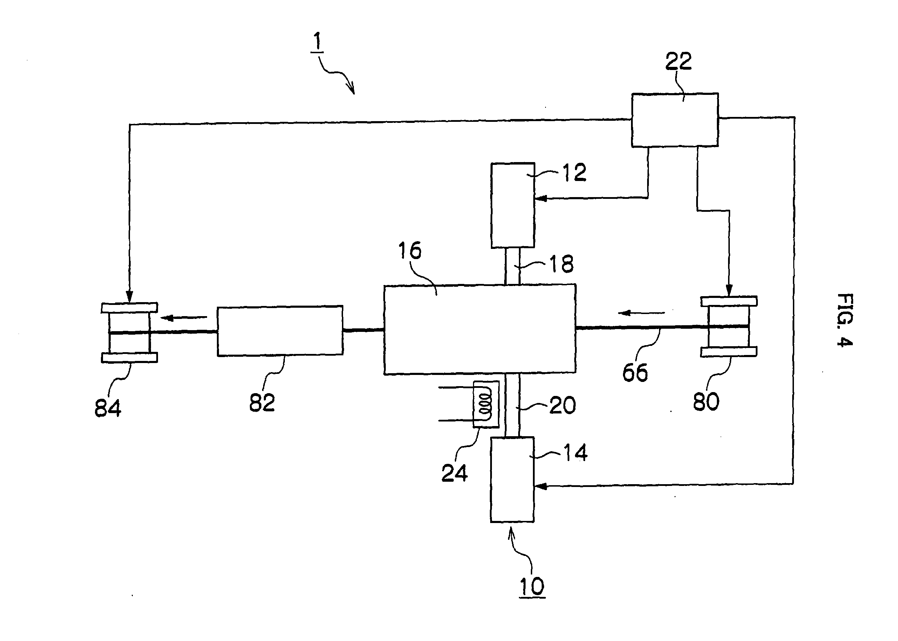

Figure 4 is a schematic view illustrating a configuration of continuous formation equipment. Continuous formation equipment 1 includes: the coating apparatus 10; a feeder 80 which feeds the coupled flexible tube assembly 66; a cooler 82 which cools the coated coupled flexible tube assembly 66; and a rewinder 84 for winding up the cooled coupled flexible tube assembly 66.

As described above, the coating apparatus 10 includes: the first extruder 12 and the second extruder 14; the crosshead 16; the first nozzle 18; the second nozzle 20 which is longer than the first nozzle 18; the control unit 22; and the heater 24 for heating the second nozzle 20.

The control unit 22 can control the feeding speeds of the feeder 80 and the rewinder 84 in addition to the supply amounts of the first extruder 12 and the second extruder 14.

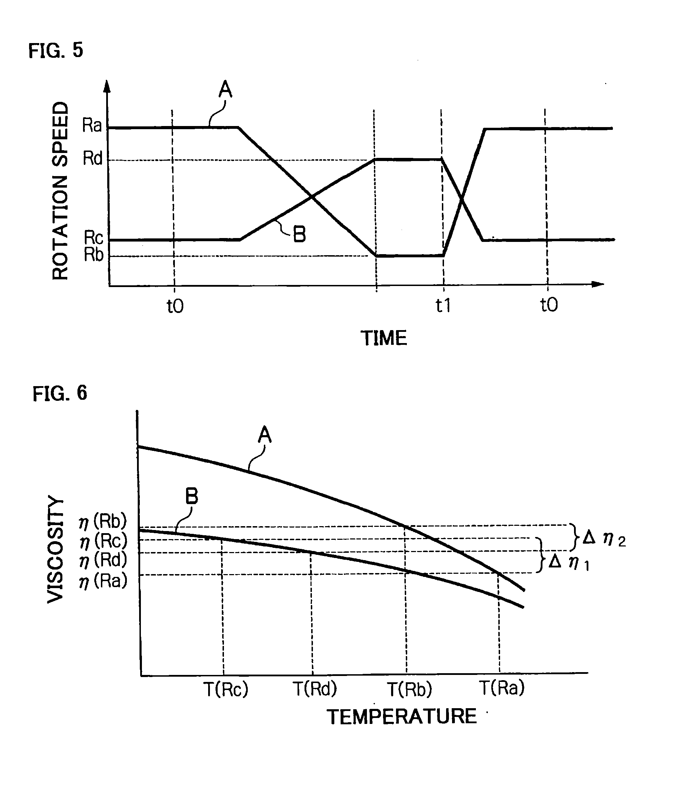

Figure 5 is a graph in which the rotation speed of the screw of each of the first extruder and the second extruder is plotted on the basis of time. As the rotation speed of the screw is higher, the amount of supplied resin becomes larger. As the rotation speed of the screw is lower, the amount of supplied resin becomes smaller. Accordingly, the rotation speed of the screw indirectly indicates the amount of supplied resin.

A line A indicates the relation between the rotation speed of the screw of the first extruder 12 and the time. This indirectly indicates the amount of the hard resin supplied from the first extruder 12. In addition, a line B indicates the relation between the rotation speed of the screw of the second extruder 14 and the time. This indirectly indicates the amount of the soft resin supplied from the second extruder 14.

t0 indicates the start time at which coating of the flexible tube assembly 64 illustrated in Figure 3 with the soft resin layer 60 and the hard resin layer 62 is started. t1 indicates the end time at which the coating of the flexible tube assembly 64 with the soft resin layer 60 and the hard resin layer 62 is ended. A period from t1 to t0 is the period during when the dummy member 68 and the joint member 70 are being coated with the soft resin layer 60 and the hard resin layer 62. That is, the sum of the length of the joint member 70 and the length of the dummy member 68 is decided according to the feeding speeds at which the feeder 80 and the rewinder 84 feed the wire member and the length of the period from the end of the above-mentioned one pattern to the start of the next pattern. Here, the feeding speed when portions corresponding to the dummy member 68 and the joint member 70 are fed to the coating apparatus 10 can be made lower than the feeding speed when a portion corresponding to the flexible tube assembly 64 is fed thereto. As a result, the portions corresponding to the dummy member 68 and the joint member 70 can be made shorter, which leads to a reduction in wasted materials.

At t0, the rotation speed (Ra) of the screw of the first extruder 12 is high, and the rotation speed (Rc) of the screw of the second extruder 14 is low. As a result, the flexible tube assembly 64 is coated with the outer coating layer 58 in which the thickness of the hard resin layer is large and the thickness of the soft resin layer is small.

Subsequently, the rotation speed of the screw of the first extruder 12 gradually becomes lower, and the rotation speed of the screw of the second extruder 14 becomes higher. Along with such changes of the rotation speeds of the screws, the thickness of the hard resin layer gradually becomes smaller, and the thickness of the soft resin layer gradually becomes larger.

At t1, the rotation speed (Rb) of the screw of the first extruder 12 is low, and the rotation speed (Rd) of the screw of the second extruder 14 is high. As a result, the flexible tube assembly 64 is coated with the outer coating layer 58 in which the thickness of the hard resin layer is small and the thickness of the soft resin layer is large.

First, with reference to Figure 6, description is given of the viscosity when a conventional soft resin and a conventional hard resin join together. Figure 6 is a graph in which the relation between the viscosity and the temperature of each of the soft resin and the hard resin is plotted. A line A indicates the relation between the viscosity and the temperature of the hard resin, and a line B indicates the relation between the viscosity and the temperature of the soft resin. Values when the rotation speed of the second extruder is Rc and Rd are used as the viscosity of the soft resin. Values when the rotation speed of the first extruder is Ra and Rb are used as the viscosity of the hard resin.

The rotation speeds when the hard resin and the soft resin join together at the beginning of the coating pattern are Ra and Rc, respectively. When the rotation speed of the first extruder 12 is Ra, the rotation speed is high, and the temperature T(Ra) of the hard resin is increased by shear heat generation inside of a barrel. The length of the first nozzle is small, and the hard resin is supplied to the crosshead at the temperature T(Ra). The viscosity at the temperature T(Ra) of the hard resin is η(Ra).

On the other hand, when the rotation speed of the second extruder 14 is Rc, the rotation speed is low, and the shear heat generation inside of the barrel is small. Therefore, the temperature T(Rc) of the soft resin is low. The length of the second nozzle is small, and the soft resin is supplied to the crosshead at the temperature T(Rc). The viscosity at the temperature T(Rc) of the soft resin is η(Rc).

The viscosity η)(Rc) of the soft resin is larger than the viscosity η(Ra) of the hard resin. Accordingly, when the soft resin and the hard resin first join together, a viscosity difference Δη1 (= η(Rc)- η(Ra)) is caused. That is, the soft resin and the hard resin join together with the viscosity difference being caused therebetween.

Next, the rotation speeds when the largest amount of the soft resin and the smallest amount of the hard resin join together at the end of the coating pattern are Rd and Rb, respectively. The rotation speed Rd of the second extruder 14 is higher than the rotation speed at the beginning of the coating pattern, and the temperature T(Rd) of the soft resin is higher than T(Rc). The length of the second nozzle is small, and hence the soft resin is supplied to the crosshead at the temperature T(Rd). The viscosity at the temperature T(Rd) of the soft resin is η(Rd). That is, the viscosity of the soft resin becomes lower than that at the beginning of the coating pattern.

On the other hand, when the rotation speed of the first extruder 12 is Rb, the rotation speed is low. Accordingly, the temperature of the hard resin becomes lower than Ra (T(Rb)). The length of the first nozzle is small, and the hard resin is supplied to the crosshead at the temperature T(Rb). The viscosity at the temperature T(Rb) of the hard resin is η(Rb). That is, the viscosity of the hard resin becomes higher than that at the beginning of the coating pattern.

When the soft resin and the hard resin join together, a viscosity difference Δη2 (=η(Rb) - η(Rd)) is caused as shown in Figure 6.

As a result of studies by the inventors of the present invention, it was found that, when the soft resin and the hard resin are caused to join together and the wire member is coated therewith, if the difference in viscosity between the soft resin and the hard resin is equal to or larger than 3,000 [Pa.s], an uneven flow is likely to be generated.

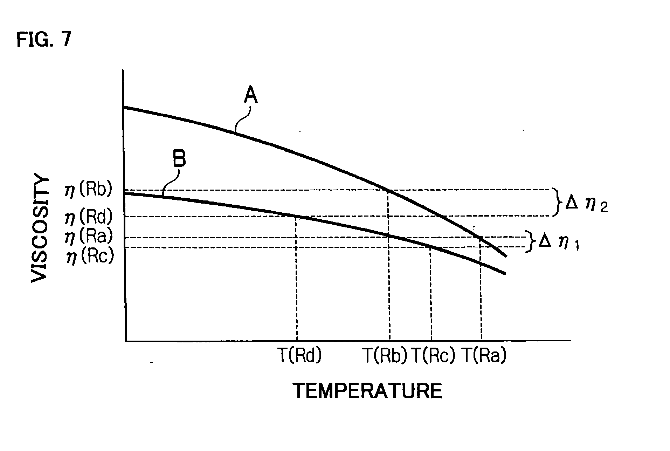

Next, description is given of the viscosity when the soft resin and the hard resin join together in the case of using the coating apparatus according to the present invention. Figure 7 is a graph in which the relation between the viscosity and the temperature of each of the soft resin and the hard resin is plotted. A line A indicates the relation between the viscosity and the temperature of the hard resin, and a line B indicates the relation between the viscosity and the temperature of the soft resin. Similarly to the above description, values when the rotation speed of the first extruder is Ra and Rb are used as the viscosity of the hard resin, and values when the rotation speed of the second extruder is Rc and Rd are used as the viscosity of the soft resin.

The rotation speeds when the hard resin and the soft resin join together at the beginning of the coating pattern are Ra and Rc, respectively. When the rotation speed of the first extruder 12 is Ra, the rotation speed is high, and the temperature T(Ra) of the hard resin in the first extruder 12 is also increased by the shear heat generation inside of the barrel. The viscosity at the temperature T(Ra) of the hard resin is η(Ra). The length of the first nozzle is small, and hence the hard resin is supplied to the crosshead 16 at the temperature T(Ra) and the viscosity η(Ra).

On the other hand, the rotation speed Rc of the second extruder 12 is low, and the temperature T(Rc) of the soft resin is low. However, in the coating apparatus of the present embodiment, the second nozzle 20 is heated by the heater 24 provided to the second nozzle 20 so as to have a temperature higher than the temperature at the exit of the second extruder 14. At this time, the rotation speed of the second extruder is as low as Rc, and hence the flow rate of the soft resin is low, so that a period of time for which the soft resin passes through the second nozzle 20 is long. In addition, the length of the second nozzle is large, and hence a heat transfer effect from the heater 24 is large. Accordingly, as shown in Figure 7, the temperature T(Rc) of the soft resin which is supplied to the crosshead 16 when the rotation speed of the second extruder is Rc is high. The viscosity η(Rc) at the temperature T(Rc) when the soft resin is supplied to the crosshead 16 is substantially the same as the viscosity η(Ra) at the temperature T(Ra) of the hard resin. That is, when the soft resin and the hard resin first join together, the soft resin and the hard resin join together with almost no viscosity difference Δη1 (= η(Rc) - η(Ra)) being caused therebetween.

Next, the rotation speeds when the largest amount of the soft resin and the smallest amount of the hard resin join together at the end of the coating pattern are Rd and Rb, respectively. When the rotation speed of the first extruder 12 is Rb, the rotation speed is lower than that at the beginning of the coating pattern, and hence the temperature T(Rb) of the hard resin is also low. The hard resin is supplied to the crosshead. The viscosity at the temperature T(Rb) of the hard resin is η(Rb). That is, the viscosity of the hard resin becomes higher than that at the beginning of the coating pattern.

On the other hand, the rotation speed Rd of the second extruder 14 is high, and the temperature T(Rd) of the soft resin is increased by the shear heat generation inside of the barrel of the extruder. The soft resin which has been extruded from the extruder passes through the second nozzle 20 to flow into the crosshead 16. At this time, the second nozzle 20 is heated by the heater 24. However, because the rotation speed Rd of the second extruder 14 is high and the flow rate of the resin is high, a period of time for which the soft resin passes through the second nozzle 20 is short. Therefore, the heat transfer effect from the heater 24 is small. Accordingly, the temperature and the viscosity of the soft resin when the soft resin passes through the second nozzle 20 to flow into the crosshead 16 are the same as the temperature and the viscosity of the soft resin at the exit of the second extruder 14. As a result, the viscosity of the soft resin flowing into the crosshead is higher when the rotation speed of the second extruder 14 is low (Rc) than when the rotation speed of the second extruder 14 is high (Rd). That is, even if the temperature of the heater 24 is constant, the amount of heat applied to the soft resin per unit time changes depending on a difference in the speed of the soft resin which passes through the second nozzle 20. With the utilization of the difference in the speed of the soft resin, it is possible to bring the viscosity of the soft resin flowing into the crosshead closer to the viscosity of the hard resin.

As shown in Figure 7, the viscosity η(Rd) at the temperature T(Rd) when the soft resin is supplied to the crosshead 16 is substantially the same as the viscosity η(Rb) at the temperature T(Rb) when the hard resin is supplied to the crosshead 16. That is, when the soft resin and the hard resin first join together, the viscosity difference Δη2 (= η(Rd) - η (Rb)) is caused. However, the difference is so small as to have no influence on an uneven flow.

Then, the inventors of the present invention found out that, when the soft resin and the hard resin are caused to join together and the wire member is coated therewith, the uneven flow can be reduced by minimizing a difference in viscosity between the two resins.

The present invention is not limited to the above-mentioned embodiment, and a large number of modified examples are possible on the basis of the above description.

For example, the soft resin may be supplied to the first extruder, and the hard resin may be supplied to the second extruder. In this example, when the hard resin passes through the second nozzle heated by the heater, a value of the viscosity thereof is adjusted. This makes it possible to reduce the difference in viscosity between the hard resin and the soft resin. Accordingly, it is possible to realize coating with a reduced uneven flow.

In addition, first, the supply amount of the soft resin may be made smaller, and the supply amount of the hard resin may be made larger. Then, the ratio thereof may be gradually changed to be reversed. That is, the present invention can be applied to both of the case where the supply amount of the soft resin is made larger first and the case where the supply amount of the hard resin is made larger first.

### Reference Signs List

1 ... continuous formation equipment, 10 ... coating apparatus, 12 first extruder, 14 ... second extruder, 16 ... crosshead, 18 ... first nozzle, 20 ... second nozzle, 22 ... control unit, 24 ... heater, 50 ... flexible tube, 58 ... outer coating layer, 60 ... soft resin layer, 62 ... hard resin layer, 64 ... flexible tube assembly, 66 ... coupled flexible tube assembly, 80 ... feeder, 82 ... cooler, 84 ... rewinder

## Claims

1. A multilayer coating apparatus which coats a wire member with a plurality of resin layers, comprising:
a first extruder which supplies a first resin;
a first nozzle which is connected to an exit of the first extruder;
a second extruder which supplies a second resin having a softening point different from a softening point of the first resin;
a second nozzle which is connected to an exit of the second extruder and is longer than the first nozzle;
a crosshead which is connected to the first nozzle and the second nozzle and coats the wire member with a first resin layer and a second resin layer;
a control unit which controls supply amounts of the first extruder and the second extruder so that a total thickness of the first resin layer and the second resin layer used for the coating is substantially constant and a ratio of a thickness of the first resin layer to a thickness of the second resin layer gradually changes; and
a heater which heats the second nozzle.

2. The multilayer coating apparatus according to claim 1, wherein the softening point of the second resin is lower than the softening point of the first resin.

3. The multilayer coating apparatus according to claim 1 or 2, further comprising
a feeder which continuously feeds the wire member to the multilayer coating apparatus, wherein:
the control device controls the first extruder and the second extruder in accordance with a predetermined pattern for gradually changing the ratio of the thickness of the first resin layer to the thickness of the second resin layer, so as to repeat the predetermined pattern a plurality of times;
the wire member at least includes:
a plurality of coating target members;
one dummy member; and
a plurality of joint members for alternately coupling the coating target member and the dummy member to each other; and
the wire member is coupled so that, one of the plurality of coating target members is fed during a period when the control device executes one predetermined pattern, and the plurality of joint members and the one dummy member are fed during a period from an end of the one predetermined pattern to a return to an initial state.

4. The multilayer coating apparatus according to any one of claims 1 to 3, wherein a length of each of the plurality of dummy members is decided in accordance with a feeding speed at which the feeder feeds the wire member and a length of the period from the end of the one pattern to the return to the initial state.

5. A multilayer coating method for coating a wire member with a plurality of resin layers, comprising:
a step of feeding the wire member to a crosshead;
a first resin supply step of supplying a first resin from a first extruder to the crosshead via a first nozzle;
a second resin supply step of supplying a second resin having a softening point different from a softening point of the first resin from a second extruder to the crosshead via a second nozzle being longer than the first nozzle;
a coating step of coating the wire member with a first resin layer and a second resin layer having a softening point different from a softening point of the first resin layer by means of the crosshead so that a total thickness of the first resin layer and the second resin layer is substantially constant and a ratio of a thickness of the first resin layer to a thickness of the second resin layer gradually changes; and
a heat step of heating the second nozzle.

6. The multilayer coating method according to claim 5, wherein the softening point of the second resin is lower than the softening point of the first resin.

7. The multilayer coating method according to claim 5 or 6, wherein the heat step includes gradually increasing an amount of heat applied to the second resin layer per unit time as the thickness of the second resin layer gradually becomes lower in the ratio.

8. The multilayer coating method according to any one of claims 5 to 7, further comprising
a wire member feed step of continuously feeding the wire member to the multilayer coating apparatus, wherein:
the wire member at least includes:
a plurality of coating target members;
one dummy member; and
a plurality of joint members for alternately coupling the coating target member and the dummy member to each other;
the coating step includes coating the plurality of coating target member according to a pattern for gradually changing the ratio of the thickness of the first resin layer to the thickness of the second resin layer so as to repeat the pattern; and
the feed step includes feeding one of the plurality of coating target members during a period when the control device executes one predetermined pattern, and feeding the plurality of joint members and the one dummy member during a period from an end of the one predetermined pattern to a return to an initial state.
